# EUROPEAN PATENT SPECIFICATION

(11) **EP 1 265 575 B1**
(45) Date of publication and mention of the grant of the patent: **20.09.2006**
(21) Application number: 00987928.9
(22) Date of filing: 20.12.2000
(51) Int. Cl.: A61F 13/532, A61F 13/474

(54) **ABSORBENT PRODUCT WITH IMPROVED SHAPING CAPACITY**
ABSORBIERENDES PRODUKT MIT VERBESSERTER FORMGEBUNGSKAPAZITÄT
PRODUIT ABSORBANT AYANT UNE CAPACITE DE MISE EN FORME AMELIOREE

(30) Priority: 29.12.1999 SE 9904835
(43) Date of publication of application: 18.12.2002
(73) Proprietor: SCA Hygiene Products AB, 405 03 Göteborg (SE)
(72) Inventor: DREVIK, Solgun, S-435 35 Mölnlycke (SE)
(74) Representative: Romare, Laila Anette
(86) International application number: PCT/SE2000/002594
(87) International publication number: WO 2001/049231

(56) References cited:
- EP-A1- 0 768 070
- EP-A1- 0 804 917
- EP-A1- 0 834 296
- WO-A1-00/02509
- WO-A1-99/32060

## Description

The invention relates to an absorbent product having a longitudinal direction and a transverse direction and comprising a liquid-permeable surface layer, a liquid impermeable surface layer, and an absorption body arranged between the surface layers, the absorption body comprising a compressible absorbent material layer.

### BACKGROUND ART

In order to capture and absorb body fluids immediately after it has left the body of a user and in order to obtain high absorption capacity in the area of an absorbent product which is expected first to be wetted by body fluids, it is common to provide absorbent products of the kind mentioned in the introduction with an absorbent raised portion. Thereby, it is important that the raised portion is not designed with a shape or size making it exert pressure or chafe against the body of the user.

By EP 0 834 296 is previously known an absorbent product comprising a layer which expands on wetting and thereby adopts a predetermined thickness profile. The known article can form a raised portion within the area of the expanding layer. A problem with such a product is to accomplish correct placement of the initially substantially flat product in relation to the body of the user. An incorrect placement may result in liquid not reaching the area of the raised portion, which means that the raised portion may not be activated and the absorption capacity thereof not utilised. In such a situation, the risk of leakage caused by poor fit and reduced absorption capability is imminent. Moreover, when the product is incorrectly positioned, the raised portion may develop in an unsuitable position in relation to the body of the user, implying an increased risk of chafing and other discomfort during use of the product.

EP 0 804917, discloses an absorbent product having an expanding layer with a multitude of incisions and apertures aiding expansion on wetting of the expanding layer. The problem with the absorbent product in EP 0 804917 is that the expanding layer produces a soft bulge with a size and shape which is determined by the amount of fluid reaching the layer. Hence, it is not possible to obtain a raised portion with a well-defined size and shape.

### DISCLOSURE OF INVENTION

There is thus still a need for a further improved absorbent product, in particular with regard to fit and leakage security which do not depend on the use situation. It is also an object of the invention to provide an absorbent product which can be produced using simple and rational production methods, and which can be packed in a compact manner without suffering as far as function is concerned.

By means of the present invention, an absorbent product of the type referred to in the introduction has been produced, which product is characterized mainly in that the compressible absorbent material layer has at least one folded portion extending in the longitudinal direction, and in that the folded portion is at least partly compressed, as a result of which the compressible absorbent material layer has a higher density within the region of the folded portion than in regions of the compressible absorbent material layer which are located outside the folded portion, the folded portion having the capacity to swell when subjected to wetting, and/or mechanical influence, and then at least partly to reassume its uncompressed shape.

According to a preferred embodiment, the folded portion extends centrally along a centre line extending in the longitudinal direction of the product.

On wetting of the absorbent product, the region of the folded portion in the compressible absorbent material layer which is subjected to wetting will absorb liquid and swell up. By arranging the compressible absorbent material layer with the folded portion facing the liquid-permeable surface layer, a raised portion will then be formed on the side of the product facing the wearer. By virtue of the fact that the raised portion is activated by wetting, this means that the absorbent product adapts its shape during use according to how it is positioned in relation to the body of the wearer, as a result of which the product is imparted a shape which is unique for each wearer. This also means that the raised portion is formed where the product is wetted, which means that the positioning of the product in the longitudinal direction is less critical. If the folded portion is instead arranged on the side of the compressible material layer which faces away from the wearer during use, the folded portion tends to expand in a direction away from the body of the wearer. As a result of this, a liquid-receiving channel facing the wearer is formed, in which liquid can be conducted away from the region which is wetted initially.

Some wearers prefer absorbent products with an essentially plane surface. According to the invention, it is possible to offer these wearers an absorbent product with a plane surface but with increased absorption capacity and improved liquid-spreading capacity within a longitudinal central portion of the product. After expansion of the compressed material fold, the material within the expanded region has a greater absorption capacity. This consequently means that, after wetting, an absorbent product according to the invention, irrespective of the direction in which the material fold in the compressible material layer faces in relation to a wearer, has increased absorption capacity within precisely that region of the product which can be expected to be wetted by the major or part of the bodily fluid discharged into the product.

In order to form a raised portion on the surface of the absorbent product which faces the wearer during use, the folded portion has a width of between 5 and 10 mm. A folded portion of this order of magnitude swells on wetting and then forms a raised portion which is sufficiently narrow to be capable of extending in a small distance between the outer labia of a female wearer without causing discomfort in the form of pressure or chafing. By virtue of the fact that the raised portion extends in between the labia, these will be parted somewhat, as a result of which liquid transfer between the body of the wearer and the product is facilitated.

In order to form edge barriers, or longitudinal edge ridges, longitudinal folds can be arranged along the side edges on the product. Such folds can be arranged so as to expand either in the direction towards the liquid-permeable surface layer or towards the liquid impermeable surface layer. When the folds are wetted and/or when they are subjected to mechanical influence, for example when the wearer moves and the product is acted on by pressure and shear forces from the legs of the wearer, the folds swell and form soft raised ridges which seal against the body of the wearer and prevent liquid from running out in the lateral direction over the edges of the product. As the folds are absorbent, they also have the capacity to absorb any liquid which reaches the edges.

A suitable material for the compressible material layer is a carded fibre layer. In this connection, a carded fibre layer consisting essentially of rayon fibres is particularly suitable.

According to one embodiment of the invention, the compressible material layer has essentially the same thickness within the region of the folded portion as within adjacent regions.

Before compression, the folded portion can have the shape of a single lobe in cross section. It is also possible to design the folded portion so that it has two or more lobes in cross section.

Furthermore, a liquid-spreading material layer can be arranged between the compressible material layer and the liquid impermeable surface layer.

According to another embodiment of the invention, an absorbent product according to the invention can comprise a soft fibrous layer made of material which collapses on wetting. Such a material layer is then arranged between the compressible material layer and the liquid-permeable surface layer. The soft fibrous layer makes the product pleasant and comfortable to wear. When the material layer collapses on wetting, the decrease in volume is compensated by a corresponding increase in volume of the compressible layer. It is consequently possible to produce an absorbent product with a surface profile which is essentially constant during use.

According to the invention, the longitudinal folded portion of the product can be arranged on a surface of the compressible material layer which faces the liquid-permeable surface layer. In such an embodiment, the fold is consequently open downwards, facing away from the body of the wearer, during use of the product. By arranging the material fold so that, during use, it expands in the direction towards the liquid-permeable surface layer and the body of the wearer, a product is obtained, which, during use, forms a raised portion shaped according to the anatomy of the wearer in that region of the material fold which is wetted by liquid.

If for any reason it is desirable to leave the surface facing the wearer essentially plane, or if only limited swelling is desired on the surface facing the wearer, the longitudinal fold formation can be arranged so as to expand in a direction towards the liquid impermeable surface layer, that is to say with the open part facing the liquid-permeable surface layer and the lobe-shaped part facing the liquid impermeable surface layer. Such a fold formation provides absorption capacity, but has no effect at all or only a slight effect on the shape of the surface of the product facing the wearer. The relationship between shaping and absorption capacity can also be controlled by virtue of the product comprising two or more folded portions which are arranged so as to expand in different directions.

It is also possible to stabilize the folded portion by binding it in the compressed state by means of a water-soluble binder. When the folded portion is wetted, the binder is dissolved and the fold can swell. The folded portion can also be stabilized by, for example, thermal bindings or other bindings which are broken when the folded portion is subjected to mechanical action. Such bindings can advantageously be used in folds arranged along the side edges of the product, where the folds is subjected to pressure and shear forces from the thighs of the wearer and where only small quantities of liquid can be expected to reach.

### BRIEF DESCRIPTION OF DRAWINGS

The invention will be described in greater detail below with reference to the exemplary embodiments shown in the appended drawings, in which
- Figure 1: shows a blank for an absorbent structure consisting of a single-folded material layer of compressible material, before compression,
- Figure 2: shows the material layer in Figure 1, after compression,
- Figure 3: shows a blank for an absorbent structure with a folded portion according to an alternative embodiment of the invention,
- Figure 4: shows a sanitary towel according to a first embodiment of the invention, comprising an absorbent structure made of compressible material,
- Figure 5: shows a section along the line V-V through the sanitary towel in Figure 4,
- Figure 6: shows a simplified longitudinal section through the sanitary towel shown in Figures 4 and 5, as it appears during use,
- Figure 7: shows a sanitary towel according to a second embodiment of the invention, which has three essentially parallel folded portions,
- Figure 8: shows a section along the line VIII-VIII through the sanitary towel in Figure 7,
- Figure 9: shows a sanitary towel according to a third embodiment of the invention,
- Figure 10: shows a cross section through a sanitary towel according to a fourth embodiment of the invention, and
- Figure 11: shows a cross section through the sanitary towel in Figure 10, as it appears after wetting.

### MODE(S) FOR CARRYING OUT THE INVENTION

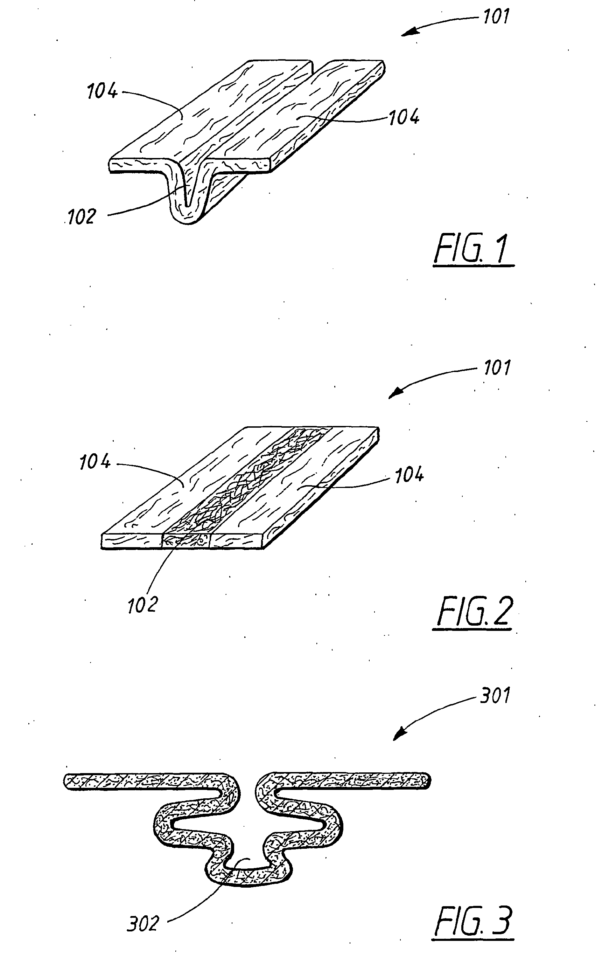

Figure 1 shows a blank 101 for an absorbent structure which is intended to be included as a component in an absorbent product, for example a sanitary towel, a panty liner, an incontinence pad or a nappy. The blank 101 consists of a compressible absorbent fibre material, a compressible absorbent foamed material or the like and has an elongate material fold 102. Suitable compressible absorbent fibre materials are, for example, fibre layers consisting essentially of cellulose fibres such as cellulose fluff pulp, cotton, rayon or the like. Carded fibrous webs of rayon fibres are particularly preferred. Suitable compressible absorbent foamed materials are absorbent cellulose foam, polyurethane foam or the like.

Figure 2 shows the blank 101 in Figure 1 after compression. The blank 101 has been compressed into an essentially plane shape. This means that the material fold 102 has been pressed together so that an elongate central strip 103 has been formed, which has a higher density than those portions 104 of the blank 101 located on either side of it.

Figure 3 shows in cross section an absorbent blank 301 in which the elongate fold has an alternative shape. The blank 301 is shown as it appears before compression and in cross section, three lobes or fold parts, as can be seen in the figure.

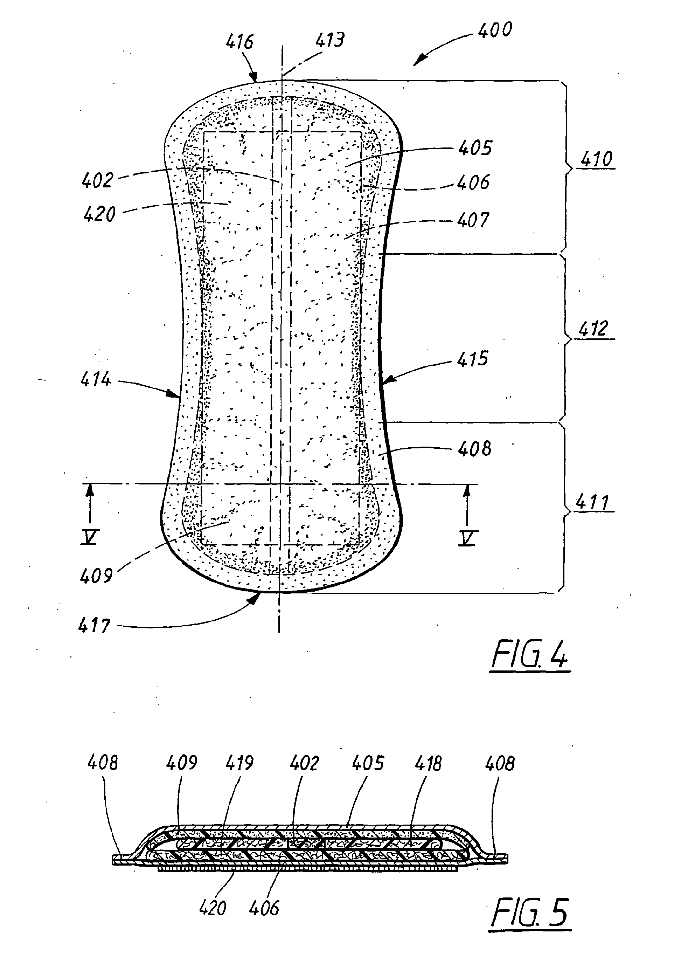

After compression, the blanks 101, 301 shown in Figures 1-3 can be used as a shaping component in an absorbent product, as is shown in Figures 4-10.

The sanitary towel 400 shown in Figures 4 and 5 has an essentially conventional construction and consequently comprises a liquid-permeable surface layer 405, a liquid impermeable surface layer 406, and an absorption body 407 enclosed between the surface layers 405, 406. The two surface layers 405, 406 are essentially the same shape as the absorption body 407, but extend a little way beyond the edges of the absorption body 407 in the plane of the sanitary towel and are connected to one another in an edge join 408 around the entire periphery of the absorption body 407. Such an edge join 408 can be brought about by, for example, gluing, sewing, or welding using heat or ultrasound.

The liquid-permeable surface layer 405 can consist of any material suitable for the purpose. Examples of commonly found liquid-permeable surface materials are various types of thin non-woven material, perforated plastic films, net material, liquid-permeable foamed material or the like. The liquid-permeable surface layer 405 can be made from two or more different materials so as to provide different functions of the surface layer. For example, it is usual to arrange a liquid-transporting layer inside a liquid-admission layer. It is also known to arrange different types of material on different parts of that surface of the sanitary towel which faces the wearer during use. Consequently, a material with good admission capacity can advantageously be arranged on that portion of the sanitary towel which is expected to be wetted first by the major part of the bodily fluid, while portions of the surface layer which are primarily intended to constitute a contact surface against the body of the wearer are provided with a material which has been optimized with regard to softness and care in relation to the skin.

The liquid impermeable surface layer 406 can also consist of any suitable liquid impermeable material. Particularly advantageous materials are thin plastic films, liquid impermeable non-woven materials, or materials which have been coated with liquid impermeable material such as wax, resin, glue or the like. It is also possible to use liquid impermeable material laminates. It may be desirable, for example, to provide the rear side of the product with an outer layer of a textile nature, for example a non-woven layer. Such a non-woven material provides a soft, skin-friendly textile surface and affords advantages such as a high degree of wearer comfort, and high friction and thus better retention against the underwear of the wearer. Moreover, a textile surface is often considered to have an aesthetically pleasing appearance. It is also often an advantage if the liquid impermeable surface layer 406 is breathable, that is to say it allows gas and water vapour to pass through the layer.

A liquid-transfer layer 409 is arranged between the liquid-permeable surface layer 405 and the absorption body 407. The liquid-transfer layer 409 fulfils a number of different functions. For example, the liquid-transfer layer 409 receives the bodily fluid discharged into the sanitary towel and then passes the liquid on to the absorption body 407 positioned below. It is therefore suitable for the liquid-transfer layer 409 to be relatively bulky with a macroporous structure which tends to receive rapidly and hold temporarily the discharged liquid. The liquid-transfer layer 409 should also have a lower density and/or be less hydrophilic than the absorption body 407, so that liquid is preferably transported in the direction from the liquid-transfer layer 409 to the absorption body 407 and not the other way. As a result of this, the liquid-transfer layer 409 also serves as an insulation layer which creates a distance between the liquid absorbed in the absorption body 407 and the body of the wearer. Moreover, the liquid-transfer layer 409 reduces the visual impression of the liquid which has been absorbed by the absorption body, which is often desirable, especially in connection with sanitary towels. Another advantage of arranging a layer between the liquid-permeable surface layer 405 and the absorption body 407 is that a liquid-transfer layer 409 often consists of a material which is airy and compressible in comparison with the absorption body 407 and is soft and flexible against the body of the wearer and as a result increases comfort and reduces the risk of chafing and other irritation of the skin of the wearer.

The sanitary towel 400 is essentially hourglass-shaped, with wider end portions 410, 411 and a narrower crotch portion 412 located between the end portions. The sanitary towel also has a centre line 413 extending in the longitudinal direction, two side edges 414, 415 extending in the longitudinal direction and curved in towards the centre line, and two end edges 416, 417 extending essentially in the transverse direction.

In the embodiment shown in Figures 4 and 5, the absorption body 407 consists of two parts 418, 419. The upper absorption part 418 is arranged immediately inside of the liquid-transfer layer 409, between the latter and the lower absorption part 419. The upper absorption part 418 is formed by a compressed blank of the type shown in Figures 1-3 and is essentially rectangular in shape with a longitudinal compressed material fold 402.

The lower absorption body 419 suitably consists of one or more layers of a material with a high absorption capacity, such as cellulose fluff pulp, absorbent bound fibre layers, tissue layers, absorbent foam, peat moss or the like. The absorption body 104 can also contain superabsorbent polymers, that is to say polymers with the capacity to absorb several times their own weight of liquid while forming a liquid-containing gel. Superabsorbents are usually in the form of particles, flakes, fibres, granules or the like. The superabsorbent material can be used on its own or together with another absorbent material. Cellulose fibre layers with a high density are a suitable material for the lower absorption part 419. In this connection, the density is preferably at least 150 g/dm³. A material with a good absorption capacity which is especially suitable for use in the lower absorption part is the dry-formed cellulose fibre material described in WO 94/10956.

In order to fix the sanitary towel in a pair of briefs, a fastening means 420 is arranged on the outside of the liquid impermeable surface layer 406 of the sanitary towel. The fastening means 420 is in the form of a longitudinal zone of self-adhesive glue. Before use, the fastening means 420 is protected in a conventional manner, for example by being covered by a protective layer of paper or plastic which has been treated with silicone or embossed so as to be easily removable from the glue when the sanitary towel is to be used. The glue can of course be arranged in any pattern suitable for the purpose. Other types of fastening means can also be used, such as friction coatings, press-studs, clips, fastening wings or the like. Different types of fastening arrangements can moreover be combined with one another. It is usual, for example, to provide a sanitary towel with both fastening glue on the rear side layer and with fastening wings.

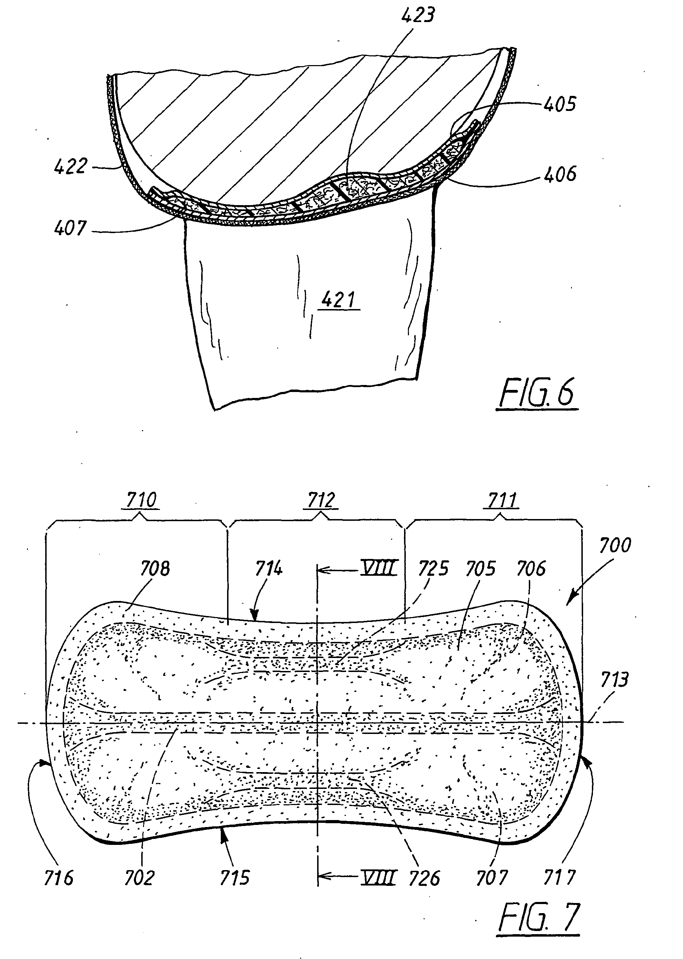

When the sanitary towel 400 is used, it is fixed in the crotch portion of the briefs of the wearer by means of the fastening means 420. Figure 6 shows a simplified longitudinal section through the sanitary towel in Figures 4 and 5, when it is arranged in the briefs 422 of a wearer 421. Consequently, only the two surface layers 405, 406, and the absorption body 407, have been drawn in the figure. For the sake of simplicity, the absorption body 407 is shown as a continuous body. Figure 6 illustrates what occurs when the sanitary towel is wetted and bodily fluid reaches the upper absorption part 418. In this connection, the bodily fluid will be absorbed by the upper absorption part 418, the latter swelling and the longitudinal material fold 402 returning at least partly to its uncompressed shape. In this way, a raised portion 423 is then formed, which has its maximum height within the primarily wetted region and which is shaped according to the body of the wearer in the wetted region. This means that the raised portion lies next to the body of the wearer 421 at the vaginal orifice and can as a result catch and absorb additional discharged bodily fluid as soon as it leaves the body of the wearer. In this way, the risk of leakage is reduced considerably. By virtue of the fact that, during use, the sanitary towel is imparted a shape adapted individually to each wearer, it is particularly comfortable to wear and has a high degree of leakage security. Furthermore, it is less critical how the sanitary towel is positioned in the briefs, and a slight displacement in the longitudinal direction is therefore of no consequence as far as the leakproofness of the sanitary towel is concerned. The invention also makes it possible for the wearer to select personally how the sanitary towel is to be arranged in the briefs. For example, in the event of night use or in other cases when the wearer is bedridden, it may be desirable to position the sanitary towel somewhat further back in the briefs than is suitable when the sanitary towel is used during the day.

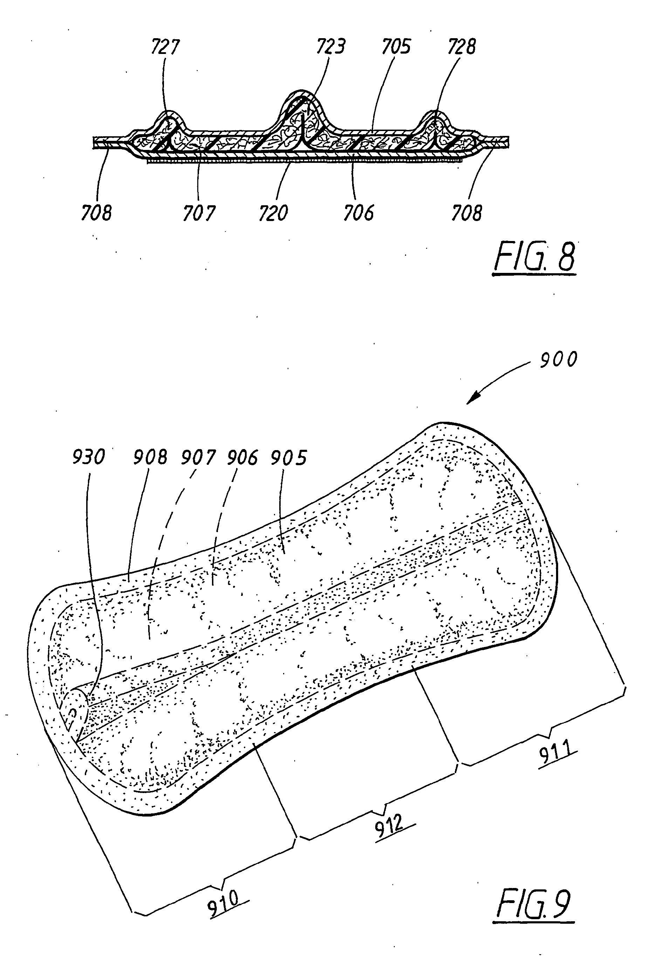

The sanitary towel 700 shown in Figures 7 and 8 is, like the sanitary towel in Figures 4-6, constructed with an absorption body 707 which is arranged between a liquid-permeable surface layer 705 and a liquid impermeable surface layer 706, which are joined together in an edge join 708.

Like the sanitary towel in Figures 4-6, the sanitary towel in Figures 7 and 8 is essentially hourglass-shaped with wider end portions 710, 711 and a narrower crotch portion 712 between the end portions 710, 711. The sanitary towel also has a longitudinal centre line 713, two longitudinal side edges 714, 715, and two transverse end edges 716, 717.

The sanitary towel 700 is also provided with a fastening means 720 for fixing the sanitary towel in the briefs of a wearer.

The sanitary towel 700 has a longitudinal central material fold 702 which extends along the centre line 713 between the end edges 716, 717 over virtually the entire length of the sanitary towel. Moreover, the sanitary towel has two additional longitudinal material folds 725, 726 which extend along the side edges 714, 715 in the crotch portion 712 of the sanitary towel. Before use, the material folds 702, 725, 726 are compressed and the sanitary towel then has an essentially plane shape. When the sanitary towel is wetted and the material folds 702, 725, 726 absorb liquid, the material folds will swell up and at least to a certain extent reassume their uncompressed appearance. As a result, a central raised portion 723 is formed in the wet region of the central fold 702 and raised ridges 727, 728 are formed along the side edges 714, 715 where liquid has reached the fold formations 725, 726 arranged along the side edges. In this way, the sanitary towel 700 is, during use, imparted a shape adapted according to the anatomy of the wearer and moreover has raised absorbent leakage barriers along the side edges 714, 715, as shown in the cross section in Figure 8. By virtue of the fact that the absorption body 707 of the sanitary towel is provided with three folded portions 702, 725, 726 within the crotch portion 712, this portion is narrower than the two end portions 710, 711 where only the central fold formation 702 is present. The folding of the absorption body 707 therefore also contributes to giving the sanitary towel a suitable anatomically correct shape in the plane of the towel.

Figure 9 shows a sanitary towel according to an embodiment of the invention in which only a part of a central longitudinal folded portion 902 has been compressed. The construction of the sanitary towel 900 is conventional, with an absorption body 907 enclosed between a liquid-permeable surface layer 905 and a liquid impermeable surface layer 906, which are connected to one another in an edge join 908. The sanitary towel 900 is hourglass-shaped and has a rear portion 910, a front portion 911 and a narrower intermediate crotch portion 912.

As can be seen from the figure, that part of the central folded portion 902 which is located within the rear portion 910 of the sanitary towel is uncompressed and forms a raised portion 930 in the rear portion 910 of the sanitary towel. Such a raised portion 930 serves as a positioning means and ensures that the wearer can position the sanitary towel correctly in the lateral direction. The raised portion 930 also constitutes protection against liquid running backwards in the groove between the body halves of the wearer.

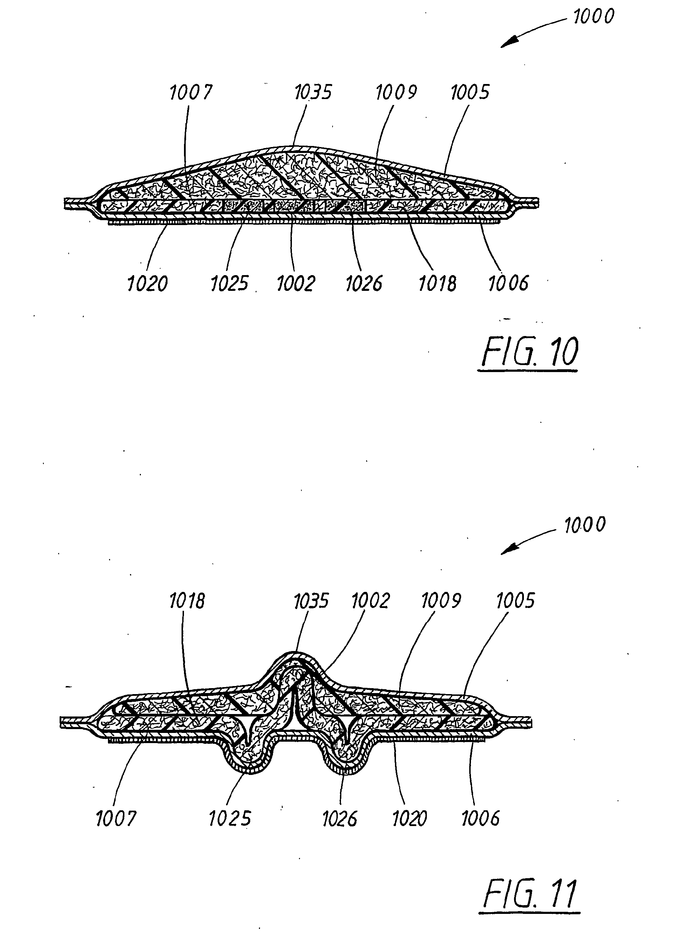

Figures 10 and 11 show in cross section a sanitary towel 1004 in which material folds in the absorption body 1007 are used in order on the one hand to impart to the sanitary towel 1004 an anatomically adapted shape and on the other hand to provide extra absorption capacity within a central region of the absorption body 1007.

Like the sanitary towels shown previously, the sanitary towel in Figures 10 and 11 comprises a liquid-permeable surface layer 1005, a liquid impermeable surface layer 1006 and an absorption body 1007 enclosed between the surface layers 1005, 1006. The sanitary towel is also provided with an adhesive fastening means 1020. The absorption body 1007 comprises a first absorbent layer 1009, and a second absorbent layer 1018 having three centrally arranged longitudinal folded porttions 1002, 1025, 1026.

As can be seen from Figure 10, the first absorbent layer 1009 is designed with a greater thickness in the central portion of the sanitary towel, as a result of which the sanitary towel has a centrally arranged longitudinal raised portion 1035. The first absorbent layer 1009 is formed from a material which collapses on wetting, as described in SE 9604226-2. The second absorbent layer 1018 has a first folded portion 1002 which, with its open part, faces the liquid impermeable surface layer 1006 and, with its lobe-shaped part, faces the liquid-permeable surface layer 1005. Arranged on either side of the first folded portion 1002 are additional folded portions 1025, 1026 which are arranged parallel to the first folded portion 1002 but which face in the opposite direction, that is to say with the open part towards the liquid-permeable surface layer 1005 and with the lobe-shaped part towards the liquidtight surface layer 1006.

When the sanitary towel 1000 in Figure 10 is subjected to wetting, on the one hand the wetted region of the first absorbent layer 1009 will collapse and lose thickness, and on the other hand the folded portions 1002, 1025, 1026 will swell. In this connection, the cross section of the sanitary towel 1000 will take on an appearance like that shown in Figure 11. Consequently, the sanitary towel will have roughly the same surface profile after wetting as it had in the dry state, because the swelling of the first folded portion 1002 coincides with a collapse of the first absorbent layer 1009, so that a central longitudinal raised portion 1035 remains on that surface of the sanitary towel which is covered by the liquid-permeable surface layer 1005.

The folded portions 1025, 1026 arranged on either side of the first folded portion 1002 swell in the direction away from the liquid-permeable surface layer 1005 and do not affect the shape of that surface of the sanitary towel facing the wearer but serve only to provide absorption capacity within that region of the sanitary towel which can be expected to be wetted first by bodily fluid.

Although the invention has been described above in connection with sanitary towels, it can of course also be applied to absorbent products such as incontinence pads, nappies and panty liners.

The various components included which have been described in connection with the different embodiments can of course be combined within the scope of the invention. Consequently, the sanitary towel shown in Figures 7 and 8 can, for example, be provided with a liquid-transfer layer and/or a collapsing layer as described in connection with Figures 4-6 and, respectively, 10 and 11. The liquid-transfer layer 409 in Figures 4-6 can be omitted, as can the collapsing layer in Figures 10 and 11. Likewise, the sanitary towel described in Figures 4-6 can be provided with a collapsing layer, and the sanitary towel in Figures 10 and 11 can be provided with a liquid-transfer layer etc. Finally, it is of course not necessary for the invention that the absorbent product is hourglass-shaped, as shown in the figures, but the invention can also be applied to absorbent products of different shapes, such as rectangular, triangular, trapezoidal, oval etc.

## Claims

1. An absorbent product having a longitudinal direction and a transverse direction, two side edges (414, 415) extending essentially in the longitudinal direction, two end edges (416, 417) extending essentially in the transverse direction, and comprising a liquid-permeable surface layer (405), a liquid impermeable surface layer (406), and an absorption body (407) arranged between the surface layers (405. 406), the absorption body (407) comprising a compressible absorbent material layer (418), **characterized in that** the compressible absorbent material layer (418) has at least one folded portion (402) extending in the longitudinal direction, and **in that** the folded portion (402) is at least partly compressed, as a result of which the compressible absorbent material layer (418) has a higher density within the region of the folded portion (402) than in regions (104) of the compressible absorbent material layer which are located outside the folded portion (402), the folded portion (402) having the capacity to swell when subjected to wetting, and/or mechanical influence, and then at least partly to reassume its uncompressed shape, wherein the folded portion (402) has a width of 5-10 mm.

2. An absorbent product according to Claim 1, in which the folded portion (402) extends centrally along a centre line (413) extending in the longitudinal direction of the product.

3. An absorbent product according to Claim 1 or 2, in which a longitudinal folded portion (725, 726) is arranged along each side edge (714, 715) on the product.

4. An absorbent product according to Claim 1, 2 or 3, in which the compressible material layer (418) consists of a carded fibre layer.

5. An absorbent product according to Claim 4, in which the carded fibre layer (418) consists essentially of rayon fibres.

6. An absorbent product according to any one of the preceding claims, in which, after compression, the compressible material layer (418) has essentially the same thickness within the region of the folded portion (402) as within adjacent regions (104).

7. An absorbent product according to any one of the preceding claims, in which, before compression, the folded portion (402) has the shape of a single lobe in cross section.

8. An absorbent product according to any one of the preceding claims, in which the folded portion (302) has two or more lobes in cross section.

9. An absorbent product according to any one of the preceding claims, in which a liquid-spreading material layer (409) is arranged between the compressible material layer (418) and the liquid impermeable surface layer (406).

10. An absorbent product according to any one of the preceding claims, in which a soft fibre layer (1009) made of material which collapses on wetting is arranged between the compressible material layer (1018) and the liquid-permeable surface layer (1005).

11. An absorbent product according to any one of the preceding claims, in which the longitudinal folded portion (402) is arranged so as to expand in the direction towards the liquid-permeable surface layer (405).

12. An absorbent product according to any one of the preceding claims, in which the longitudinal folded portion (1025, 1026) is arranged so as to expand in the direction towards the liquid impermeable surface layer (406).

13. An absorbent product according to any one of the preceding claims, in which the product comprises folded portions (1002, 1025, 1026) which are arranged so as to expand in different directions.

14. An absorbent product according to any one of the preceding claims, in which the folded portion (402) is bound by a water-soluble binder.

## Patentansprüche

1. Absorbierendes Produkt mit einer Längsrichtung und einer Querrichtung, zwei Seitenkanten (414, 415), die sich im Wesentlichen in der Längsrichtung erstrecken, zwei Endkanten (416, 417), die sich im Wesentlichen in der Querrichtung erstrecken, und umfassend eine flüssigkeitsdurchlässige Oberflächenlage (405), eine flüssigkeitsundurchlässige Oberflächenlage (406) und einen Absorptionskörper (407), der zwischen den Oberflächenlagen (405, 406) angeordnet ist, wobei der Absorptionskörper (407) eine komprimierbare Lage aus Absorptionsmaterial (418) umfasst, **dadurch gekennzeichnet, dass** die komprimierbare Lage aus Absorptionsmaterial (418) wenigstens einen gefalteten Abschnitt (402) aufweist, der sich in Längsrichtung erstreckt, und **dadurch**, dass der gefaltete Abschnitt (402) wenigstens teilweise komprimiert ist, wodurch die komprimierbare Lage aus Absorptionsmaterial (418) im Bereich des gefalteten Abschnitts (402) eine höhere Dichte aufweist als in anderen Bereichen (104) der komprimierbaren Lage aus Absorptionsmaterial, die außerhalb des gefalteten Abschnitts (402) liegen, wobei der gefaltete Abschnitt (402) die Fähigkeit aufweist zu quellen, wenn er einer Benetzung und/oder einem mechanischen Einfluss ausgesetzt wird und dann wenigstens teilweise seine unkomprimierte Form wieder annimmt, wobei der gefaltete Abschnitt (402) eine Breite von 5-10 mm aufweist.

2. Absorbierendes Produkt nach Anspruch 1, bei dem sich der gefaltete Abschnitt (402) mittig entlang einer Mittellinie (413), die sich in Längsrichtung des Produkts erstreckt, erstreckt.

3. Absorbierendes Produkt nach Anspruch 1 oder 2, bei dem ein längslaufender gefalteter Abschnitt (725, 726) entlang jeder Seitenkante (714, 715) des Produkts angeordnet ist.

4. Absorbierendes Produkt nach Anspruch 1, 2 oder 3, bei dem die komprimierbare Materiallage (418) aus einer kardierten Faserlage besteht.

5. Absorbierendes Produkt nach Anspruch 4, bei dem die kardierte Faserlage (418) im Wesentlichen aus Rayonfasern besteht.

6. Absorbierendes Produkt nach einem der vorstehenden Ansprüche, bei dem nach der Kompression die komprimierbare Materiallage (418) innerhalb des Bereichs des gefalteten Abschnitts (402) im Wesentlichen die gleiche Stärke aufweist wie in benachbarten Bereichen (104).

7. Absorbierendes Produkt nach einem der vorstehenden Ansprüche, bei dem der gefaltete Abschnitt (402) vor der Kompression im Querschnitt die Form einer einzelnen Nocke aufweist.

8. Absorbierendes Produkt nach einem der vorstehenden Ansprüche, bei dem der gefaltete Abschnitt (302) im Querschnitt zwei oder mehrere Nocken aufweist.

9. Absorbierendes Produkt nach einem der vorstehenden Ansprüche, bei dem eine Flüssigkeitsverteilungslage (409) zwischen der komprimierbaren Materiallage (418) und der flüssigkeitsundurchlässigen Oberflächenlage (406) angeordnet ist.

10. Absorbierendes Produkt nach einem der vorstehenden Ansprüche, bei dem eine weiche Faserlage (1009), die aus einem Material gebildet ist, das auf eine Benetzung kollabiert, zwischen der komprimierbaren Materiallage (1018) und der flüssigkeitsdurchlässigen Oberflächenlage (105) angeordnet ist.

11. Absorbierendes Produkt nach einem der vorstehenden Ansprüche, bei dem der längslaufende gefaltete Abschnitt (402) derart angeordnet ist, dass er sich in der Richtung zur flüssigkeitsdurchlässigen Oberflächenlage (405) ausdehnt.

12. Absorbierendes Produkt nach einem der vorstehenden Ansprüche, bei dem der längslaufende gefaltete Abschnitt (1025, 1026) derart angeordnet ist, dass er sich in der Richtung zur flüssigkeitsundurchlässigen Oberflächenlage (406) ausdehnt.

13. Absorbierendes Produkt nach einem der vorstehenden Ansprüche, bei dem das Produkt gefaltete Abschnitte (1002, 1025, 1026) umfasst, die derart angeordnet sind, dass sie sich in unterschiedlichen Richtungen ausdehnen.

14. Absorbierendes Produkt nach einem der vorstehenden Ansprüche, bei dem der gefaltete Abschnitt (402) durch ein wasserlösliches Bindemittel begrenzt ist.

## Revendications

1. Produit absorbant ayant une direction longitudinale et une direction transversale, deux bords latéraux (414, 415) s'étendant essentiellement dans la direction longitudinale, deux bords d'extrémité (416, 417) s'étendant essentiellement dans la direction transversale, et comprenant une couche de surface perméable aux liquides (405), une couche de surface imperméable aux liquides (406), et un corps d'absorption (407) disposé entre les couches de surface (405, 406), le corps d'absorption (407) comprenant une couche de matériau absorbant compressible (418), **caractérisé en ce que** la couche de matériau absorbant compressible (418) comporte au moins une partie pliée (402) s'étendant dans la direction longitudinale, et **en ce que** la partie pliée (402) est au moins partiellement comprimée, en résultat de quoi la couche de matériau absorbant compressible (418) a une densité plus élevée à l'intérieur de la région de la partie pliée (402) que dans les régions (104) de la couche de matériau absorbant compressible qui sont situées à l'extérieur de la partie pliée (402), la partie pliée (402) ayant la capacité de gonfler lorsqu'elle est soumise au mouillage, et/ou à une influence mécanique, puis de reprendre au moins partiellement sa forme non-comprimée, la partie pliée (402) ayant une largeur comprise entre 5 et 10 mm.

2. Produit absorbant selon la revendication 1, dans lequel la partie pliée (402) s'étend de façon centrale le long d'une ligne centrale (413) s'étendant dans la direction longitudinale du produit.

3. Produit absorbant selon la revendication 1 ou 2, dans lequel une partie pliée longitudinale (725, 726) est disposée sur le long de chaque bord latéral (714, 715) sur le produit.

4. Produit absorbant selon la revendication 1, 2 ou 3, dans lequel la couche de matériau compressible (418) est constituée par une couche de fibres cardées.

5. Produit absorbant selon la revendication 4, dans lequel la couche de fibres cardées (418) est constituée essentiellement par des fibres de rayonne.

6. Produit absorbant selon l'une quelconque des revendications précédentes, dans lequel, après compression, la couche de matériau absorbant compressible (418) a essentiellement la même épaisseur à l'intérieur de la région de la partie pliée (402) qu'à l'intérieur des régions adjacentes (104).

7. Produit absorbant selon l'une quelconque des revendications précédentes, dans lequel, avant compression, la partie pliée (402) a en section transversale la forme d'un lobe unique.

8. Produit absorbant selon l'une quelconque des revendications précédentes, dans lequel la partie pliée (302) comporte, en section transversale, deux ou plusieurs lobes.

9. Produit absorbant selon l'une quelconque des revendications précédentes, dans lequel une couche de matériau diffusant les liquides (409) est disposée entre la couche de matériau compressible (418) et la couche de surface imperméable aux liquides (406).

10. Produit absorbant selon l'une quelconque des revendications précédentes, dans lequel une couche de fibres molles (1009) réalisée en un matériau qui s'écrase lors du mouillage est disposée entre la couche de matériau compressible (1018) et la couche perméable aux liquides (1005).

11. Produit absorbant selon l'une quelconque des revendications précédentes, dans lequel la partie pliée longitudinale (402) est agencée de façon à se dilater dans la direction vers la couche de surface perméable aux liquides (405).

12. Produit absorbant selon l'une quelconque des revendications précédentes, dans lequel la partie pliée longitudinale (1025, 1026) est agencée de façon à se dilater dans la direction vers la couche de surface imperméable aux liquides (406).

13. Produit absorbant selon l'une quelconque des revendications précédentes, dans lequel le produit comprend des parties pliés (1002, 1025, 1026) qui sont agencées de façon à se dilater dans des directions différentes.

14. Produit absorbant selon l'une quelconque des revendications précédentes, dans lequel la partie pliée (402) est fixée par un agent de fixation soluble dans l'eau.
